(19) Europäisches Patentamt
European Patent Office
Office européen des brevets

(11) EP 4 186 489 A1

(12) # EUROPEAN PATENT APPLICATION

(43) Date of publication:
**31.05.2023 Bulletin 2023/22**

(21) Application number: **21210711.4**

(22) Date of filing: **26.11.2021**

(51) International Patent Classification (IPC):
***A61K 8/368*** *(2006.01)* ***A61K 8/39*** *(2006.01)*
***A61K 8/44*** *(2006.01)* ***A61K 8/46*** *(2006.01)*
***A61Q 19/00*** *(2006.01)* ***A61Q 19/10*** *(2006.01)*
***C11D 1/00*** *(2006.01)* ***C11D 1/94*** *(2006.01)*
***C11D 3/20*** *(2006.01)*

(52) Cooperative Patent Classification (CPC):
**A61K 8/368; A61K 8/39; A61K 8/44; A61K 8/466; A61Q 19/00; A61Q 19/10; C11D 1/94;**
A61K 2800/596; C11D 1/28

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Kao Corporation**
**Chuo-ku**
**Tokyo 103-8210 (JP)**

(72) Inventors:
- **Weitzel, Johanna**
**64297 Darmstadt (DE)**
- **Burgdorf, Kristin**
**64297 Darmstadt (DE)**

(74) Representative: **Hoffmann Eitle**
**Patent- und Rechtsanwälte PartmbB**
**Arabellastraße 30**
**81925 München (DE)**

(54) **PERSONAL CLEANSING COMPOSITION COMPRISING ALPHA-SULFO FATTY ACID SURFACTANTS**

(57) The present invention is directed to an aqueous personal cleansing composition comprising a) one or more amphoteric and/or zwitterionic surfactant(s) and/or their salt(s), b) one or more non-ionic surfactant(s), c) one or more alpha-sulfo fatty acid surfactant(s) and/or their salt(s), d) one or more non-sulfate anionic surfactant(s) and/or their salt(s), different from the ones according to group c).

EP 4 186 489 A1

## Description

### Field of the invention

The present invention relates to an aqueous personal cleansing composition and method for cleansing of keratin substrates.

### Background of the invention

Modern consumers desire cleansing products for their body and hair, which provide multiple benefits beyond cleansing. For example, mildness of the compositions is highly desired as well as environmental friendliness. Additionally, cleansing products should protect the color of artificially-colored hair from premature fading.

Cosmetic industry has tackled these problems by developing cleansing products comprising surfactant mixtures.

EP2902010 discloses a newly synthesized surfactant class, namely alpha-sulfofattyacid salts in combination with alkyl glucosides.

EP2902011 discloses alpha-sulfofattyacid salts in combination with amidoalkyl betaine surfactants.

EP3246384 discloses alpha-sulfofattyacid salts in combination with N-acyl amino acid surfactants.

Despite the attempts of the prior art, there is a real need to develop cleansing compositions with improved mildness, color protection, and foaming performance. The prior art has not sufficiently tackled these problems.

### Summary of the invention

The first object of the present invention is an aqueous personal cleansing composition comprising:

a) one or more amphoteric and/or zwitterionic surfactant(s) and/or their salt(s),

b) one or more non-ionic surfactant(s),

c) one or more alpha-sulfo fatty acid surfactant(s) and/or their salt(s),

d) one or more non-sulfate anionic surfactant(s) and/or their salt(s), different from the ones according to group c).

The second object of the present invention is a method of cleansing keratin substrates, preferably human keratin substrates, more preferably the human body or hair, comprising the steps of:

i) applying the composition as defined above onto the keratin substrate and leaving it for a time period in the range of 10 s to 600 s,

ii) rinsing-off the keratin substrates with water,

iii) optionally drying the keratin substrates.

The third object of the present invention is a use of the composition as defined above for improving wash fastness of artificially-colored hair.

### Detailed description of the invention

Inventors of the present invention have unexpectedly found out that a composition according to claim 1 had superior foaming performance and color protection on dyed keratin fibers. Hence, wash fastness of the artificially-colored hair was improved. The composition further exhibited mildness to the skin and hair, resulting in a soft feel of the keratin substrates.

### Cleansing composition

The present invention is directed to an aqueous personal cleansing composition comprising:

a) one or more amphoteric and/or zwitterionic surfactant(s) and/or their salt(s),

b) one or more non-ionic surfactant(s),

c) one or more alpha-sulfo fatty acid surfactant(s) and/or their salt(s),

d) one or more non-sulfate anionic surfactant(s) and/or their salt(s), different from the ones according to group c).

It is preferred from the viewpoint of consumer convenience that the composition of the present invention is a hair shampoo composition.

The term 'aqueous' within the meaning of the present invention denotes a composition with a total concentration of water of 50% by weight or more, preferably of 60% by weight or more, more preferably of 70% by weight or more, calculated to the total weight of the composition.

It is preferred from the viewpoint of cosmetic safety and mildness that the pH of the composition is 3 or more, more preferably the pH is 4 or more, still more preferably the pH is 4.5 or more.

It is preferred from the viewpoint of cosmetic safety and mildness that the pH of the composition is 9 or less, more preferably the pH is 8 or less, still more preferably the pH is 7 or less.

For attaining the above-mentioned effects, it is preferred that the pH of the composition is in the range of 3 to 9, preferably in the range of 4 to 8, more preferably in the range of 4.5 to 7.

It is preferred from the viewpoint of foaming performance and mildness that one or more compound(s) according to group a) is/are one or more alkyl betaine surfactant(s), one or more alkyl amidoalkyl betaine surfactant(s), one or more alkyl amphoacetate surfactant(s), one or more sultaine surfactant(s), one or more hydroxy sultaine surfactant(s), and/or their salt(s), and/or their mixtures,

Suitable amphoteric/zwitterionic surfactants may be selected from compounds according to the general structure(s)

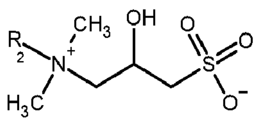

and/or

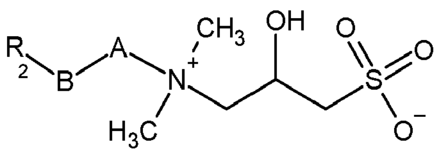

wherein $R_2$ is a straight or branched, saturated or unsaturated, substituted or unsubstituted alkyl chain with a carbon number of $C_{10}$ to $C_{22}$, preferably $R_2$ is a straight alkyl chain with a carbon number of $C_{10}$ to $C_{16}$, A is a straight alkyl chain with a carbon number of $C_1$ to $C_6$ or a branched alkyl chain with a carbon number of $C_3$ to $C_6$, preferably A is a linear alkyl chain with a carbon number of $C_3$, and B is an amide or an ester group.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine, and/or their salt(s).

Further suitable amphoteric/zwitterionic surfactants are of betaine type. Suitable compounds may be selected from alkyl betaines and/or alkylamido betaines.

It is preferred from the viewpoint of mildness that one or more compound(s) according to group a) is/are coco betaine, cocoamidopropyl betaine, coco amphoacetate, and/or their salt(s), and/or their mixtures, more preferably it is cocoamidopropyl betaine and/or its salt(s).

It is preferred from the viewpoint of cleansing and foaming performance that the total concentration of compound(s) according to group a) is 1 % by weight or more, preferably 2% by weight or more, more preferably 3% by weight or more, calculated to the total weight of the composition.

It is preferred from the viewpoint of mildness that the total concentration of compound(s) according to group a) is 20% by weight or less, preferably 15% by weight or less, more preferably 12% by weight or less, calculated to the total weight of the composition.

For attaining the above-mentioned effects, it is preferred that the total concentration of compound(s) according to group a) is in the range of 1 % to 20% by weight, preferably in the range of 2% to 15% by weight, more preferably in the range of 3% to 12% by weight, calculated to the total weight of the composition.

Preferably, from the viewpoint of foaming performance and wash fastness one or more compound(s) according to group b) is/are one or more alkyl glucoside(s), one or more alkyl polyglycoside(s), one or more ethoxylated and/or propoxylated fatty alcohol(s), one or more ethoxylated and/or propoxylated vegetable oil(s), one or more N-alkylpolyhydroxyalkylamide surfactants.

Suitable nonionic surfactants are alkyl polyglycosides according to the general structure:

$$R_3O(R_4O)_tZ_x$$

Wherein Z denotes a carbohydrate with $C_5$ to $C_6$, $R_3$ is an alkyl group with $C_8$ to $C_{18}$, $R_4$ is methyl, ethyl or propyl, t ranges from 0 to 10, and x ranges from 1 to 5. Suitable compounds according to this structure are $C_9$-$C_{11}$ alkylpolyglycoside, the structures disclosed in EP-A 70 074, and JP 2015-123019A.

The preferred compounds according to the structure of above are decyl glucoside, lauryl glucoside, and coco glucoside, and the most preferred one is decyl glucoside.

Suitable examples for non-ionic surfactants are fatty alcohol ethoxylates of the following general structure

$$R_5\ (OCH_2CH_2)_{n1}\ OH$$

wherein $R_5$ is straight or branched, saturated or unsaturated alkyl chain which may be synthetic or natural with a C chain length in the range of 8 to 40, preferably 9 to 30 and more preferably 9 to 24 and $n_1$ is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactants are polypropylene glycol ethers of fatty alcohol according to general structure

$$R_6\ (OCH_2\text{-}CH_2\text{-}CH_2)_{n2}\ OH$$

wherein $R_6$ is straight or branched, saturated or unsaturated fatty alcohol which may be synthetic or natural with a C chain length in the range of 8 to 40, preferably 9 to 30 and more preferably 9 to 24 and $n_2$ is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

$$R_7\ C(O)\ (OCH_2CH_2)_{n3}\ OH$$

wherein $R_7$ is straight or branched, saturated or unsaturated alkyl group which may be synthetic or natural with a C chain length in the range of 7 to 39, preferably 9 to 29 and more preferably 9 to 23 and $n_3$ is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate,

PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

$$R_8\ C(O)\ (OCH_2\text{-}CH_2\text{-}CH_2)_{n4}\ OH$$

wherein $R_8$ is straight or branched, saturated or unsaturated alkyl group which may be synthetic or natural with a C chain length in the range of 7 to 39, preferably 9 to 29 and more preferably 9 to 23 and $n_4$ is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are polyethylene glycol and polypropylene glycol ether of fatty alcohols of the following general structure

$$R_9(OCH_2\text{-}CH_2\text{-}CH_2)_{n5}(OCH_2CH_2)_{n6}OH$$

wherein $R_9$ is straight or branched, saturated or unsaturated alkyl group which may be synthetic or natural with a C chain length in the range of 7 to 39, preferably 9 to 29 and more preferably 9 to 23 and $n_5$ and $n_6$ may be the same or different and are a number in the range of 1 to 40.

Further suitable nonionic surfactants are ethoxylated vegetable oils. Well-known and commonly used examples are ethoxylated castor oil such as PEG-40 hydrogenated castor oil or and PEG-60 hydrogenated castor oil.

The preferred compound(s) according to group b) is/are one or more alkyl glycoside, more preferably one or more $C_8$-$C_{22}$ alkyl glucoside, from the viewpoint of wash fastness and mildness. The most preferred compound(s) according to group b) is/are coco glucoside, decyl glucoside, and lauryl glucoside.

It is preferred from the viewpoint of wash fastness that the total concentration of one or more compound(s) according to group b) is/are 0.25% by weight or more, more preferably 0.5% by weight or more, still more preferably 0.75% by weight or more, calculated to the total weight of the composition.

It is preferred from the viewpoint of mildness that the total concentration of one or more compound(s) according to group b) is/are 15% by weight or more, more preferably 12% by weight or more, still more preferably 10% by weight or more, calculated to the total weight of the composition.

For attaining the above-mentioned effects, it is preferred that the total concentration of one or more compound(s) according to group b) is/are in the range of 0.25% to 15% by weight, preferably in the range of 0.5% to 12% by weight, more preferably in the range of 0.75% to 10% by weight, calculated to the total weight of the composition.

The composition comprises one or more alpha-sulfo fatty acid surfactant as compound(s) according to group c).

It is preferred from the viewpoint of commercial availability that one or more compound(s) according to group c) is one or more compound(s) according to the following general structure

$$R_1CH(SO_3M_1)COOM_2$$

in which the radical $R_1$ is a linear or branched alkyl or alkenyl chain with 6 to 16 carbon atoms and the radicals $M_1$ and $M_2$ are independently of one another selected from the group H, Li, Na, K, Ca/2, Mg/2, ammonium and/or alkanolamine, preferably $R_1$ is selected from saturated, linear alkyl chain with 10 to 16 carbon atoms, more preferably one or more compound(s) according to group c) is disodium sulfolaurate.

It is preferred from the viewpoint of mildness and wash fastness that the total concentration of one or more compound(s) according to group c) is 0.1 % by weight or more, more preferably 0.25% by weight or more, still more preferably 0.5% by weight or more, calculated to the total weight of the composition.

It is preferred from the viewpoint of mildness that the total concentration of one or more compound(s) according to group c) is 10% by weight or less, more preferably 8% by weight or less, more preferably 5% by weight or less, calculated to the total weight of the composition.

For attaining the above-mentioned effects, it is preferred that the total concentration of one or more compound(s) according to group c) is in the range of 0.1 % to 10% by weight, preferably in the range of 0.25% to 8% by weight, more preferably in the range of 0.5% to 5% by weight, calculated to the total weight of the composition.

The composition of the present invention comprises one or more non-sulfate anionic surfactant as compound(s) according to group d).

The term 'non-sulfate' within the meaning of the present invention denotes a surfactant, which does not carry sulfate groups.

It is preferred from the viewpoint of mildness that one or more compound(s) according to group d) is/are one or more sarcosinate surfactant(s), one or more glutamate surfactant(s), one or more isethionate surfactant(s), one or more ether sulfonate surfactant(s), one or more sulfosuccinate surfactant(s), one or more sulfoacetate surfactant(s), one or more glycinate surfactant(s), one or more alaninate surfactant(s), one or more carboxylate surfactant(s), one or more taurate surfactant(s), and/or their mixtures, and/or their salt(s).

The more preferred surfactant according to group d) is/are one or more sarcosinate surfactant(s).

Suitable compounds are lauroyl sarcosinate, cocoyl sarcosinate, myristyl sarcosinate, oleyl sarcosinate, and/or their mixtures, and/or their salts, in particular their sodium, potassium and/or triethanolamine salts.

It is preferred from the viewpoint of wash fastness and mildness that the total concentration of one or more compound(s) according to group d) is 0.1% by weight or more, more preferably 0.25% by weight or more, still more preferably 0.5% by weight or more, calculated to the total weight of the composition.

It is preferred from the viewpoint of mildness that the total concentration of one or more compound(s) according to group d) is 10% by weight or less, more preferably 8% by weight or less, still more preferably 5% by weight or less, calculated to the total weight of the composition.

For attaining the above-mentioned effects, it is preferred that the total concentration of one or more compound(s) according to group d) is in the range of 0.1 % to 10% by weight, preferably in the range of 0.25% to 8% by weight, more preferably in the range of 0.5% to 5% by weight, calculated to the total weight of the composition.

It is preferred from the viewpoint of mildness that the total concentration of sulfate-based surfactants in the composition of the present invention is 2% by weight or less, preferably 1% by weight or less, more preferably 0.5% by weight or less, still more preferably 0.1% by weight or less, calculated to the total weight of the composition, still more preferably the composition is free of sulfate-based surfactants.

It is further preferred from the viewpoint of wash fastness that the weight ratio of compound(s) according to group a) to compound(s) according to group c) is 1 or more, preferably 1.5 or more, more preferably 2 or more.

This, suitable weight ratios of compound(s) a) to c) are in the range of 1:1 to 10:1, preferably in the range of 1.5:1 to 8:1, more preferably in the range of 2:1 to 7:1.

It is further preferred from the viewpoint of mildness that the total concentration of compounds a), b), c), and d) is 30% by weight or less, preferably 25% by weight or less, more preferably 20% by weight or less, still more preferably 15% by weight or less, calculated to the total weight of the composition.

It is further preferred from the viewpoint of cleansing performance that the total concentration of compounds a), b), c), and d) is 3% by weight or more, preferably 5% by weight or more, more preferably 7% by weight or more, still more preferably 10% by weight or more, calculated to the total weight of the composition.

It is further preferred from the viewpoint of mildness that the composition of the present invention comprises one or more cationic or amphoteric polymer, preferably selected from Polyquaternium-6, Polyquaternium-10, Polyquaternium-16, Polyquaternium-47, Polyquaternium-53, and/or their salt(s) and/or their mixtures.

Suitable concentration ranges for the cationic or amphoteric polymers are in the range of 0.01% to 3% by weight, preferably in the range of 0.1% to 2% by weight, more preferably in the range of 0.15% to 1.5% by weight, calculated to the total weight of the composition.

Thus, the disclosure of the present invention also relates to an aqueous personal cleansing composition, preferably a hair shampoo composition, comprising:

a) one or more alkyl amidoalkyl betaine surfactant(s) and/or their salt(s),

b) one or more alkyl glycoside,

c) one or more alpha-sulfo fatty acid surfactant(s) and/or their salt(s),

d) one or more sarcosinate anionic surfactant(s) and/or their salt(s), different from the ones according to group c).

The disclosure of the present invention also relates to an aqueous personal cleansing composition, preferably a hair shampoo composition, comprising:

a) one or more alkyl amidoalkyl betaine surfactant(s) and/or their salt(s),

b) one or more alkyl glycoside,

c) one or more alpha-sulfo fatty acid surfactant(s) and/or their salt(s),

d) one or more sarcosinate anionic surfactant(s) and/or their salt(s), different from the ones according to group c),

wherein the weight ratio of compound(s) according to group a) to compound(s) according to group c) is 1 or more, preferably 1.5 or more, more preferably 2 or more.

The disclosure of the present invention is also directed to an aqueous personal cleansing composition, preferably a hair shampoo composition, comprising:

a) one or more alkyl amidoalkyl betaine surfactant(s) and/or their salt(s) at a total concentration in the range of 3% to 12% by weight, calculated to the total weight of the composition,

b) one or more alkyl glycoside at a total concentration in the range of 0.75% to 10% by weight, calculated to the total weight of the composition,

c) one or more alpha-sulfo fatty acid surfactant(s) and/or their salt(s) at a total concentration in the range of 0.5% to 5% by weight, calculated to the total weight of the composition,

d) one or more sarcosinate anionic surfactant(s) and/or their salt(s), different from the ones according to group c), at a total concentration in the range of 0.5% to 5% by weight, calculated to the total weight of the composition.

The disclosure of the present invention is also directed to an aqueous personal cleansing composition, preferably a shampoo composition, comprising:

a) one or more alkyl amidoalkyl betaine surfactant(s) and/or their salt(s) at a total concentration in the range of 3% to 12% by weight, calculated to the total weight of the composition,

b) one or more alkyl glycoside at a total concentration in the range of 0.75% to 10% by weight, calculated to the total weight of the composition,

c) one or more alpha-sulfo fatty acid surfactant(s) and/or their salt(s) at a total concentration in the range of 0.5% to 5% by weight, calculated to the total weight of the composition,

d) one or more sarcosinate anionic surfactant(s) and/or their salt(s), different from the ones according to group c), at a total concentration in the range of 0.5% to 5% by weight, calculated to the total weight of the composition,

wherein the weight ratio of compound(s) according to group a) to compound(s) according to group c) is 1 or more, preferably 1.5 or more, more preferably 2 or more.

**Method of cleansing**

The present invention is also directed to a method of cleansing keratin substrates, preferably human keratin substrates, more preferably the human body or hair, comprising the steps of:

i) applying the composition as defined above onto the keratin substrate and leaving it for a time period in the range of 10 s to 600 s,

ii) rinsing-off the keratin substrates with water,

iii) optionally drying the keratin substrates.

**Use of the composition**

The present invention is also directed to a use of the composition as defined above for improving wash fastness of artificially-colored hair.

It is preferred from the viewpoint of wash fastness improvement that the hair was artificially-colored with hair direct dyes.

The following examples are to illustrate the invention, but not to limit it.

**EXAMPLES**

**Table 1**

| Ingredients | | Inv. Ex. 1 | Inv. Ex. 2 | Inv. Ex. 3 | Comp. Ex. 1 |
|---|---|---|---|---|---|
| | | [% by weight] | | | |
| a) | Cocoamidopropyl betaine | 6.0 | 4.0 | 6.0 | 11.0 |
| b) | Coco glucoside | 5.0 | 5.0 | 3.0 | - |
| c) | Disodium 2-sulfolaurate | 2.0 | 2.0 | 3.0 | 2.0 |
| d) | Sodium lauroyl sarcosinate | 2.0 | 4.0 | 3.0 | 2.0 |
| - | Water | 100.0 | | | |
| Wash fastness | | | | | |
| - | ΔE* | 5.05 | 5.60 | 5.28 | 7.32 |

The inventive compositions exhibited a better wash fastness in comparison to the comparative composition.

**Methods**

**Hair dyeing**

Human hairstreaks (21 cm, 2 g per bundle) were bleached twice with a commercial hair bleach available under the trade name Goldwell Silklift. After bleaching, the streaks were permed with a commercial perming product available under the trade name Goldwell Topform Type 1 for 20 min, rinsed, and then treated with an oxidative composition for 10 min for perm fixing. The hair streaks were shampooed and dried.

The hair was dyed with an oxidative hair dye composition comprising direct dyes available under the tradename Goldwell Topchic 6R after mixing with 6% by weight of hydrogen peroxide in a weight ratio of 1:1 to prepare ready-to-use compositions having a pH in the range of 10.0 to 10.5. The hair streaks were dyed for 30 min at room temperature and then rinsed off with water. After drying the hair streaks, hair color was measured with a color-difference meter (Datacolor Mercury Check II Plus) in the CIE colorimetric system (L*, a*, b*).

**Wash fastness**

10 g of the compositions above were diluted with 90 g water. The dyed hairstreaks were immersed in the diluted shampoo composition at 40°C and placed on a shaking bath having a shaking frequency of 100 rpm for 30 min. The hairstreaks were then handwashed for 1 min under lukewarm water. This process was repeated for 4 times. After the repetitive treatment, the hairstreaks were blow-dried and the remaining color was measured (L*, a*, b*). ΔE* was calculated as the color difference between 5 times shaking bath treatment ($L^*_0$, $a^*_0$, $b^*_0$) and freshly colored hair ($L^*_1$, $a^*_1$, $b^*_1$).

$$\Delta E^* = \sqrt{(L_1^* - L_0^*)^2 + (a_1^* - a_0^*)^2 + (b_1^* - b_0^*)^2}$$

The following examples are within the scope of the present invention.

**EXAMPLE 4**

| | % by weight |
|---|---|
| Coco betaine | 8.0 |
| Decyl glucoside | 3.0 |
| Disodium 2-sulfolaurate | 4.0 |
| Potassium lauroyl sarcosinate | 1.0 |
| Polyquaternium-53 | 0.5 |

(continued)

| | % by weight |
|---|---|
| Glyceryl oleate | 0.5 |
| Sodium chloride | 0.5 |
| NaOH/HCl | ad pH 5.5 |
| Water | ad 100.0 |

**EXAMPLE 5**

| | % by weight |
|---|---|
| Cocoamidopropyl hydroxysultaine | 6.0 |
| Lauryl glucoside | 3.0 |
| Disodium 2-sulfolaurate | 4.0 |
| Capryleth-9 carboxylic acid | 1.0 |
| Polyquaternium-10 | 0.5 |
| Glyceryl oleate | 0.5 |
| Sodium chloride | 0.5 |
| NaOH/HCl | ad pH 5.5 |
| Water | ad 100.0 |

## Claims

1. An aqueous personal cleansing composition comprising:

    a) one or more amphoteric and/or zwitterionic surfactant(s) and/or their salt(s),
    b) one or more non-ionic surfactant(s),
    c) one or more alpha-sulfo fatty acid surfactant(s) and/or their salt(s),
    d) one or more non-sulfate anionic surfactant(s) and/or their salt(s), different from the ones according to group c).

2. The composition according to claim 1 **characterized in that** the total concentration of water is 50% by weight or more, preferably 60% by weight or more, more preferably 70% by weight or more, calculated to the total weight of the composition.

3. The composition according to any of the claims 1 and/or 2 **characterized in that** the pH of the composition is in the range of 3 to 9, preferably in the range of 4 to 8, more preferably in the range of 4.5 to 7.

4. The composition according to any of the preceding claims **characterized in that** one or more compound(s) according to group a) is/are one or more alkyl betaine surfactant(s), one or more alkyl amidoalkyl betaine surfactant(s), one or more alkyl amphoacetate surfactant(s), one or more sultaine surfactant(s), one or more hydroxy sultaine surfactant(s), and/or their salt(s), and/or their mixtures, preferably it is coco betaine, cocoamidopropyl betaine, coco amphoacetate, and/or their salt(s), and/or their mixtures, more preferably it is cocoamidopropyl betaine and/or its salt(s).

5. The composition according to any of the preceding claims **characterized in that** the total concentration of compound(s) according to group a) is in the range of 1% to 20% by weight, preferably in the range of 2% to 15% by weight, more preferably in the range of 3% to 12% by weight, calculated to the total weight of the composition.

6. The composition according to any of the preceding claims **characterized in that** one or more compound(s) according to group b) is/are one or more alkyl glucoside(s), one or more alkyl polyglycoside(s), one or more ethoxylated and/or propoxylated fatty alcohol(s), one or more ethoxylated and/or propoxylated vegetable oil(s), one or more N-alkylpolyhydroxyalkylamide surfactants, and/or their mixtures, preferably it is one or more alkyl glycoside, more preferably one or more C8-C22 alkyl glucoside, still more preferably it is coco glucoside and/or decyl glucoside, and/or their

mixtures.

7. The composition according to any of the preceding claims **characterized in that** the total concentration of one or more compound(s) according to group b) is/are in the range of 0.25% to 15% by weight, preferably in the range of 0.5% to 12% by weight, more preferably in the range of 0.75% to 10% by weight, calculated to the total weight of the composition.

8. The composition according to any of the preceding claims **characterized in that** one or more compound(s) according to group c) is one or more compound according to the following general structure

$$R_1CH(SO_3M_1)COOM_2$$

in which the radical $R_1$ is a linear or branched alkyl or alkenyl chain with 6 to 16 carbon atoms and the radicals $M_1$ and $M_2$ are independently of one another selected from the group H, Li, Na, K, Ca/2, Mg/2, ammonium and/or alkanolamine, preferably $R_1$ is selected from saturated, linear alkyl chain with 10 to 16 carbon atoms, more preferably one or more compound(s) according to group c) is disodium sulfolaurate.

9. The composition according to any of the preceding claims **characterized in that** the total concentration of one or more compound(s) according to group c) is in the range of 0.1% to 10% by weight, preferably in the range of 0.25% to 8% by weight, more preferably in the range of 0.5% to 5% by weight, calculated to the total weight of the composition.

10. The composition according to any of the preceding claims **characterized in that** one or more compound(s) according to group d) is/are one or more sarcosinate surfactant(s), one or more glutamate surfactant(s), one or more isethionate surfactant(s), one or more ether sulfonate surfactant(s), one or more sulfosuccinate surfactant(s), one or more sulfoacetate surfactant(s), one or more glycinate surfactant(s), one or more alaninate surfactant(s), one or more carboxylate surfactant(s), one or more taurate surfactant(s), and/or their mixtures, and/or their salt(s), preferably it is one or more sarcosinate surfactant(s), more preferably it is sodium lauroyl sarcosinate.

11. The composition according to any of the preceding claims **characterized in that** the total concentration of one or more compound(s) according to group d) is in the range of 0.1 % to 10% by weight, preferably in the range of 0.25% to 8% by weight, more preferably in the range of 0.5% to 5% by weight, calculated to the total weight of the composition.

12. The composition according to any of the preceding claims **characterized in that** the total concentration of sulfate-based surfactants is 2% by weight or less, preferably 1% by weight or less, more preferably 0.5% by weight or less, still more preferably 0.1% by weight or less, calculated to the total weight of the composition, still more preferably the composition is free of sulfate-based surfactants.

13. The composition according to any of the preceding claims **characterized in that** it comprises one or more cationic or amphoteric polymer, preferably selected from Polyquaternium-6, Polyquaternium-10, Polyquaternium-16, Polyquaternium-47, Polyquaternium-53, and/or their salt(s) and/or their mixtures.

14. The composition according to any of the preceding claims **characterized in that** the weight ratio of compound(s) according to group a) to compound(s) according to group c) is 1 or more, preferably 1.5 or more, more preferably 2 or more.

15. A method of cleansing keratin substrates, preferably human keratin substrates, more preferably the human body or hair, comprising the steps of:

    i) applying the composition as defined in any of the claims 1 to 14 onto the keratin substrate and leaving it for a time period in the range of 10 s to 600 s,
    ii) rinsing-off the keratin substrates with water,
    iii) optionally drying the keratin substrates.

16. A use of the composition as defined in any of the claims 1 to 14 for improving wash fastness of artificially-colored hair.

Europäisches Patentamt
European Patent Office
Office européen des brevets

# EUROPEAN SEARCH REPORT

Application Number
EP 21 21 0711

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 16 August 2021 (2021-08-16), anonymous: "Nourishing Shampoo", XP055918037, Database accession no. 8938567 * the whole document * | 1,4,6,8, 10,12, 13,15,16 | INV. A61K8/368 A61K8/39 A61K8/44 A61K8/46 A61Q19/00 A61Q19/10 C11D1/00 C11D1/94 C11D3/20 |
| X | ANONYMOUS: "Absolute Beautifying Shampoo", GNPD, July 2015 (2015-07), XP002761817, [retrieved on 2015-07-01] * the whole document * | 1,4,6,8, 10,12, 15,16 | |
| X | EP 2 468 842 B1 (JOHNSON & JOHNSON CONSUMER INC [US]) 14 December 2016 (2016-12-14) * paragraphs [0001], [0007], [0008], [0028], [0031] - [0033]; claims; examples * | 1-16 | |
| | | | TECHNICAL FIELDS SEARCHED (IPC) |
| Y | US 2017/007523 A1 (MAX EVA [DE] ET AL) 12 January 2017 (2017-01-12) * paragraphs [0001], [0009], [0035], [0038]; claims; examples * | 1-16 | A61K C11D A61Q |
| Y | CN 104 622 766 B (GUANGZHOU KENENG COSMETIC RES CO LTD) 1 September 2017 (2017-09-01) * the whole document * | 1-16 | |
| Y | US 2021/299020 A1 (CRUZ LUIS ALBERTO [MX] ET AL) 30 September 2021 (2021-09-30) * paragraphs [0001], [0004], [0005], [0010], [0013], [0016], [0053]; claims; examples * | 1-16 | |

The present search report has been drawn up for all claims

2

| Place of search | Date of completion of the search | Examiner |
|---|---|---|
| Munich | 5 May 2022 | Heller, Dorothée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 21 0711

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2022

| Patent document cited in search report | | Publication date | Patent family member(s) | | | Publication date |
|---|---|---|---|---|---|---|
| EP 2468842 | B1 | 14-12-2016 | AU | 2011250841 | A1 | 05-07-2012 |
| | | | BR | PI1106007 | A2 | 09-04-2013 |
| | | | CA | 2762192 | A1 | 21-06-2012 |
| | | | CN | 102552063 | A | 11-07-2012 |
| | | | EP | 2468842 | A1 | 27-06-2012 |
| | | | KR | 20120070513 | A | 29-06-2012 |
| | | | RU | 2011152104 | A | 27-06-2013 |
| | | | US | 2012157365 | A1 | 21-06-2012 |
| US 2017007523 | A1 | 12-01-2017 | AR | 099237 | A1 | 06-07-2016 |
| | | | BR | 112016018065 | B1 | 26-01-2021 |
| | | | CN | 105934236 | A | 07-09-2016 |
| | | | EP | 2902010 | A1 | 05-08-2015 |
| | | | ES | 2667168 | T3 | 09-05-2018 |
| | | | JP | 6585629 | B2 | 02-10-2019 |
| | | | JP | 2017505342 | A | 16-02-2017 |
| | | | KR | 20160115979 | A | 06-10-2016 |
| | | | PL | 2902010 | T3 | 31-07-2018 |
| | | | US | 2017007523 | A1 | 12-01-2017 |
| | | | US | 2018338898 | A1 | 29-11-2018 |
| | | | WO | 2015117840 | A1 | 13-08-2015 |
| CN 104622766 | B | 01-09-2017 | NONE | | | |
| US 2021299020 | A1 | 30-09-2021 | US | 2021299020 | A1 | 30-09-2021 |
| | | | WO | 2021202400 | A1 | 07-10-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2902010 A
- EP 2902011 A
- EP 3246384 A
- EP 70074 A
- JP 2015123019 A